(19) Europäisches Patentamt
European Patent Office
Office européen des brevets

(11) Veröffentlichungsnummer: **0 003 014**
**B1**

# (12) EUROPÄISCHE PATENTSCHRIFT

(45) Veröffentlichungstag der Patentschrift: **14.07.82**

(51) Int. Cl.³: **C 07 C 31/08**, C 07 C 31/10, C 07 C 31/12, C 07 C 29/04

(21) Anmeldenummer: **79200002.8**

(22) Anmeldetag: **03.01.79**

(54) **Verfahren zur Herstellung von Alkoholen mit 2-4 C-Atomen durch katalytische Hydratation von Olefinen.**

(43) Veröffentlichungstag der Anmeldung:
**11.07.79 Patentblatt 79/14**

(45) Bekanntmachung des Hinweises auf die Patenterteilung:
**14.07.82 Patentblatt 82/28**

(84) Benannte Vertragsstaaten:
**BE DE FR GB IT NL**

(56) Entgegenhaltungen:
**DE-A-1 618 999**
**FR-A-1 560 386**
**FR-A-2 324 601**
**GB-A-727 721**
**US-A-2 050 445**

**CHEMICAL ENGINEERING, Band 79, no. 19, september 4, 1972, Albany, New York, USA R. DEVON et al.: »Ethanol via Direct Hydratation«, Seiten 50 und 51.**

(73) Patentinhaber: **CHEMISCHE WERKE HÜLS AG, Postfach 1320, D-4370 Marl 1 (DE)**

(72) Erfinder: **Sommer, August, Dr., Birnenbruchstrasse 10, D-4690 Herne 1 (DE)**
Erfinder: **Heitmann, Wilhelm, Dr., Bahnhofstrasse 7 b, D-4690 Herne 1 (DE)**
Erfinder: **Brücker, Rainer, Dr., Unterspredey 65, D-4620 Castrop-Rauxel (DE)**

(74) Vertreter: **Steil, Hanna, Dipl.-Chem. et al, RSP PATENTE-PB 40 Holsterhauser Strasse 160 Postfach 2840, D-4690 Herne 2 (DE)**

EP 0 003 014 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Verfahren zur Herstellung von Alkoholen mit 2—4 C-Atomen durch katalytische Hydratation von Olefinen

## Beschreibung und Beispiele

Es ist bekannt, aliphatische Alkohole, insbesondere Äthylalkohol, Isopropylalkohol, sek. und tertiären Butylalkohol, dadurch synthetisch herzustellen, daß man Olefine, das heißt, insbesondere Äthylen, Propylen oder Butene zusammen mit Wasser bei erhöhter Temperatur und erhöhtem Druck über saure Katalysatoren leitet. Als Katalysator kommt vor allem Phosphorsäure, die in porösen Trägern aufgesogen ist, oder Ionentauscher in der H-Form in Betracht. Es ist ferner bekannt, daß neben der gewünschten Reaktion zur Alkoholbildung unter Reaktionsbedingungen auch Nebenreaktionen des Olefins mit anderen Olefinmolekülen oder mit Wasser oder mit beidem ablaufen, die neben einer Verunreinigung des Hauptproduktes auch eine Verringerung der Ausbeute bewirken, da die Nebenprodukte vom Hauptprodukt abgetrennt und beseitigt werden müssen. Solche Nebenprodukte sind vor allem Kohlenwasserstoffe, die aus der Zusammenlagerung mehrerer Olefinmoleküle entstehen, Äther aus zwei Olefinmoleküle unter Reaktion mit einem Wassermolekül, andere Alkohole als gewünscht, die entweder Isomere des gewünschten Alkohols sind oder höhere Alkohole, die durch Reaktion von solchen Olefinen mit Wasser entstehen, die sich erst durch Oligomerisation des Ausgangsolefins gebildet haben, und schließlich auch Aldehyde und Ketone, die durch Dehydrierung aus den gebildeten Alkoholen noch unter Reaktionsbedingungen entstehen.

Der Menge nach überwiegen die Äther unter den entstehenden Nebenprodukten, gefolgt von Kohlenwasserstoffen und unerwünschten Nebenprodukt-Alkoholen, während die Aldehyde und Ketone mengenmäßig am unbedeutendsten sind. Im übrigen ist der mengenmäßige Anfall von Nebenprodukten stark von den Reaktionsbedingungen, nämlich Temperatur, Druck, Kontaktverweilzeit und Molverhältnis Wassser : Olefin, abhängig.

Im Prinzip ist zu erwarten, daß auch die Bildung von Nebenprodukten durch chemische Reaktionen erfolgt, die in beiden Richtungen ablaufen und der schließlich sich einstellende Umsatz bei ausreichend langer Kontaktverweilzeit von den Gleichgewichtskonzentrationen der Reaktionspartner unter Reaktionsbedingungen und der Ausgangskonzentration abhängig ist. Ausgenommen sind davon solche Nebenprodukte, die sicher irreversibel entstehen, wo also ein oder mehrere Endprodukte nicht mehr für das Gleichgewicht zur Verfügung stehen. Dies trifft beispielsweise zu für Oligomerisationsprodukte des Olefins mit einem so hohen Molekulargewicht, daß unter Reaktionsbedingungen kein nennenswerter Dampfdruck mehr vorhanden ist.

Es trifft aber auch für die Bildung der Aldehyde und Ketone zu, weil der bei der Dehydrierung entstehende Wasserstoff mit dem im großen Überschuß vorhandenen Olefin unter Bildung des Paraffins reagieren kann, so daß er als Gleichgewichtspartner ausscheidet.

Für beide Verbindungsgruppen — hochpolymerisierte Kohlenwasserstoffe sowie Aldehyde und Ketone — wurde für die Fälle der direkten Gasphasenhydration von Wasser mit Äthylen oder Propylen zu Äthylalkohol oder Isopropylalkohol experimentell nachgewiesen, daß sie nicht als Produkte einer Gleichgewichtsreaktion entstehen. Bei Zugabe dieser Stoffe zum Einsatzgas zum Reaktor in der Größenordnung der Menge, die unter Reaktionsbedingungen als Nebenprodukt entsteht, zeigte sich nämlich, daß die Nebenproduktbildung in dieser Beziehung nicht zurückgedrückt werden kann, sondern daß sich vielmehr die auftretende Gesamtmenge dieser Stoffe additiv aus der üblicherweise unter den jeweiligen Reaktionsbedingungen entstehenden Menge und der zugegebenen Menge zusammensetzt. Ausbeuteverluste in dieser Richtung können also zwar bis zu einem gewissen Umfang durch geeignete Wahl der Reaktionsbedingungen verringert, nicht jedoch ganz vermieden werden. Aldehyde und Ketone haben ein ähnliches Löslichkeitsverhalten in Wasser wie die entsprechenden Alkohole und werden so mit den produzierten Alkoholen aus dem Umlaufolefin entfernt, so daß sich dort keine höheren Konzentrationen aufbauen. Aus dem produzierten Alkohol müssen dann natürlich bei entsprechenden Reinheitsanforderungen die Aldehyde und Ketone entfernt werden. Die höhermolekularen Kohlenwasserstoffe verbleiben bei der Alkoholabtrennung zum großen Teil im Umlaufolefin und werden sinnvollerweise, wie in DT-C-1 768 207 und DT-C-1 960 139 dargestellt, durch eine destillative Behandlung eines Teils des Umlaufolefins abgetrennt, um Umsatzverluste und mögliche Belegungen der Apparatur durch Produkte weitergehender Polymerisation zu vermeiden.

Es ist seit langem bekannt, daß die den jeweiligen Alkoholen entsprechenden Äther in einer Gleichgewichtsreaktion entstehen. Ohne in die komplizierten Theorien der Ätherentstehung eintreten zu wollen, kann man sich die Bildung rein stöchiometrisch am einfachsten als Weiterreaktion des gebildeten Alkohols mit einem zusätzlichen Olefin-Molekül vorstellen, z. B.

$$C_2H_5OH + C_2H_4 \rightarrow (C_2H_5)_2O$$

oder

$$C_3H_7OH + C_3H_6 \rightarrow (C_3H_7)_2O.$$

Alle drei Reaktionspartner, d. h. Alkohol, Olefin und Äther stehen dem Gleichgewicht zur Verfügung. Sie gehören nicht zu den vorgehend erwähnten Stoffen, d. h. hochpolymerisierten Kohlenwasserstoffen und Aldehyden bzw. Ketonen, die nicht dem Gleichgewicht zur Verfügung stehen, weil entweder der Dampfdruck zu niedrig ist oder wegen des erheblichen Überschusses eines Reaktionspartners eine praktisch quantitative Weiterreaktion erfolgt.

So wurde bereits in US-A-2 050 445 für die Äthanolherstellung in einem kontinuierlichen Verfahren mit wäßriger Phosphorsäure als Katalysator davon berichtet (Seite 3, linke Spalte, Zeile 44—54), daß durch Zurückführen von Diäthyläther mit dem Umlaufgas die weitere Bildung von Äther zurückgedrückt werden kann. Die Rückführung von Diisopropyläther zum Umlaufgas der Isopropylalkohol-Synthese aus Propylen ist in der CA-A-867 797 beschrieben. Es wird also bereits seit längerer Zeit zur Erhöhung der Ausbeute in den Äthanol- und Isopropylalkohol-Synthesen der entsprechende Äther in der erzeugten Menge zum Umlaufgas zurückgegeben.

Die Ausbeute an gewünschtem Alkohol wird aber noch durch weitere Nebenprodukte, d. h. niederpolymerisierte Kohlenwasserstoffe und unerwünscht entstehende Alkohole, verringert, die außerdem zusätzliche Reinigungsdestillationen erfordern.

Gemäß DE-A-2 301 208 wurde erstmals bei der Herstellung von Rohalkohol — einem ca. 10%igen Alkohol — versucht, die Nebenprodukte außer Äther nicht zu verwerfen, sondern zumindest zum Teil die Bildung auch der weiteren Nebenprodukte neben Äther durch Rückführung derselben zu unterbinden bzw. zu verringern. Man erreicht dies dadurch, daß man die in der Waschextraktionskolonne nach Abtrennung des Olefins in wäßriger Lösung anfallenden Nebenprodukte rückführte. Dies geht dort aus Sp. 4, Zeile 65 ff und der Zeichnung hervor. Es wurde also nach Art und Menge allenfalls möglich gehalten, die wasserlöslichen Nebenprodukte rückzuführen, die Rückführung der organischen Phase wurde nicht in Betracht gezogen, sondern diese wasserunlöslichen Nebenprodukte wurden über einen Seitenabzug (Leitung 22) aus dem System entfernt. Zudem wurde nur unter milden Reaktionsbedingungen gearbeitet, was zwangsläufig einen geringeren Umsatz, verbunden mit erhöhten Energiekosten, bedeutet.

Es war daher Aufgabe der Erfindung einen Weg zu finden, die Bildung der gesamten anfallenden Nebenprodukte zu unterbinden.

Es wurde nun überraschend gefunden, daß man — auch bei Arbeiten unter schärferen Bedingungen — die Gesamtmenge an Nebenprodukten, die in der Waschextraktion als auch noch bei der Aufkonzentrierung anfallen, rückführen kann, ohne daß sich betriebliche Störungen ergeben.

Dies war nicht naheliegend, da man annehmen mußte, daß eine derart hohe Menge nicht mehr zu einer Verringerung der Nebenproduktbildung führt, insbesondere im Hinblick auf die unerwünschten Alkohole nach der Aufkonzentrierung.

Gegenstand der Erfindung ist daher ein Verfahren zur Herstellung von Alkoholen mit 2—4 C-Atomen durch katalytische Hydratation der entsprechenden Olefine an sauren Katalysatoren bei erhöhter Temperatur und erhöhtem Druck, dadurch gekennzeichnet, daß dem Einsatzprodukt vor Eintritt in den Reaktor die Nebenprodukte der Hydratation größenordnungsmäßig in der Menge zugeführt werden, in der sie beim Durchgang des Einsatzproduktes durch das Katalysatorbett entstehen und in wäßriger und organischer Phase anfallen.

Im wesentlichen handelt es sich bei der Hydratation von Äthylen um die Nebenprodukte Diethyläther, Oligomere des Äthylens, Butanole und Hexanole, bei der Hydratation von Propylen um Diisopropyläther, Oligomere des Propylens, n-Propanol, Hexanole, Methylpentanole und Nonanole.

Bei den als Nebenprodukt entstehenden unerwünschten Alkoholen, insbesondere auch erst nach der Aufkonzentrierung, die also entweder Isomere des gewünschten Produktes sind oder durch Wasseranlagerung an Oligomerisationsprodukten des Ausgangsolefins entstanden sind, war es von der Theorie her erheblich unsicher, ob eine Zugabe, auch insbesondere eine hohe Zugabe, dieser Stoffe zum Umlaufgas zur gewünschten Zurückdrängung ihrer Bildung führen würde. Bei den sich von den Oligomerisationsprodukten des Ausgangsolefins ableitenden Alkoholen ergibt sich die Unsicherheit daher, daß zwei aufeinanderfolgende, unterschiedliche Reaktionen zu ihrer Bildung erforderlich sind und es durchaus fraglich erscheint, ob beide Rückreaktionen unter den vorliegenden Reaktionsbedingungen mit der erforderlichen Geschwindigkeit verlaufen, um am Ende in der Bilanz das nicht weitere Entstehen dieser Verbindungen auswerfen zu können.

Noch unwahrscheinlicher war jedoch, ob auch bei mit den zum gewünschten Alkohol isomeren Verbindungen die Zurückdrängung der Bildung durch Zugabe zum Einsatzolefin möglich ist. Diese Isomeren, z. B. n-Propanol und n-Butanol, entstehen in kleinen Mengen abweichend von der Regel von Markownikow, nach der Isopropylalkohol und sec. Butanol entstehen müßten. Nur für diese Hydratation gemäß der Regel von Markownikow ist bei der durch Protonen katalysierten Reaktion ein Mechanismus vorstellbar, beispielsweise

$$CH_2{=}CH-CH_3 \longleftrightarrow CH_2^{\ominus}-C^{\oplus}H-CH_3$$

$$CH_2^{\ominus}-C^{\oplus}H-CH_3 + H^{\oplus} \rightarrow CH_3-C^{\oplus}H-CH_3$$

$$CH_3-C^{\oplus}H-CH_3 + O^{\ominus}H \rightarrow CH_3-CHOH-CH_3$$

Hierbei reagiert also zunächst das Proton mit dem Carbeniat-Kohlenstoffatom, das in der polaren Grenzstruktur des Olefins vorliegt. Bei dieser überschaubaren Reaktion ist in Gegenwart von Säure die Umkehrung vorstellbar, so daß kein Zweifel daran besteht, daß es sich um eine Gleichgewichtsreaktion handelt, was für die Isomeren, die abweichend von der Regel von Markownikow entstehen, nicht zutrifft.

Bei den niedermolekularen Kohlenwasserstoffen als Produkt weniger Oligomerisationsstufen ist theoretisch eher mit einer Gleichgewichtsreaktion zu rechnen, etwa

$$C_2H_4 + C_2H_4 \rightarrow C_4H_8, \quad C_4H_8 + C_2H_4 \rightarrow C_6H_{12}$$

$$C_3H_6 + C_3H_6 \rightarrow C_6H_{12}, \quad C_6H_{12} + C_3H_6 \rightarrow C_9H_{18}$$

Da diese niedermolekularen Kohlenwasserstoffe sich bei der Destillation in Gegenwart von Wasserdampf ähnlich wie die Äther verhalten, führt man sie auf einfache Weise wieder zurück, wenn man eine Reinigung des aus dem Rohalkohol durch hydroselektive Destillation abgetrennten Rohäthers vor der Rückgabe zum Einsatzolefin unterläßt. Dabei ist lediglich durch entsprechende Fahrweise der in DE-C-1 768 207 und DE-C-1 960 139 beschriebenen Umlaufgasreinigung darauf zu achten, daß eventuell mit zurückgeführte höhermolekulare Kohlenwasserstoffe ausgeschleust und die niedermolekularen im Umlaufgas belassen werden.

Bei den rückzuführenden Verbindungen handelt es sich um die entsprechenden Äther, niedermolekularen Kohlenwasserstoffe aus der Oligomerisation des Ausgangsolefins und alle als Nebenprodukt entstehenden Alkohole, und zwar sowohl Isomere des gewünschten Hauptproduktes als auch Hydratationsprodukte der Oligomeren des Ausgangsolefins. Da angenommen wird, daß alle genannten Verbindungen sich in Gleichgewichtsreaktionen bilden, muß die dem Umlaufgas zugesetzte Menge nur in der Größenordnung der unter Reaktionsbedingungen erzeugten Menge entsprechen. Insbesondere bei Änderung der Reaktionsbedingungen ist bis zum Auftreten der Nebenprodukte in der Destillation deren entstandene Menge nicht genau bekannt. Wenn geringfügig höhere Mengen der Nebenprodukte zugegeben werden, als der augenblicklichen Bildung entspricht, stellt sich für alle genannten Stoffe auch die Gleichgewichtskonzentration ein, bilanziell wird dann Nebenprodukt in Olefin bzw. Olefin und Wasser zurückverwandelt.

Es ist sowohl möglich, alle genannten Nebenprodukte gleichzeitig in der größenordnungsmäßig gebildeten Menge dem Umlaufgas wieder zuzugeben als auch einzelne wegzulassen und nur die anderen zurückzugeben. Dies ist insbesondere dann von Bedeutung, wenn einige Nebenprodukte anderweitig wirtschaftlich verwertbar sind, wie dies beispielsweise bei Diäthyläther häufig der Fall ist.

Neben einem Anheben der Ausbeute des Olefins an gewünschtem Alkohol wird durch die Erfindung die destillative Reinigung des anfallenden Rohalkohols erheblich vereinfacht. So entfällt eine Nachreinigung des als obere Schicht des Kopfproduktes der Hydroselektion anfallenden Äthers, und auch der die Nebenproduktalkohole enthaltende Seiten Abzug aus der Alkoholrektifikation braucht beim Wiedereinsatz in der Hydratation weder vom drain enthaltenden Hauptproduktalkohol noch vom Wasser befreit zu werden, da Wasser ohnehin als Rohstoff zugegeben wird und kleine Mengen zurückgegebenes Hauptprodukt zwar geringfügig den Umsatz, nicht jedoch wie beim Verwerfen die wirtschaftlich wesentlichere Ausbeute nachteilig beeinflussen.

Die Reaktionsbedingungen bei der Hydratation der Olefine liegen in folgenden Bereichen:

Hydratation in der Gasphase:
- Äthanol:
  - 200—300° C, 30—100 bar
  - Wasser/Olefin-Verhältnis 0,2 : 1 bis 1 : 1
- Isopropanol:
  - 150—220° C, 10—50 bar
  - Wasser/Olefin-Verhältnis 0,2 : 1 bis 0,8 : 1
- Butanole:
  - 150—220° C, 10—50 bar
  - Wasser-Olefin-Verhältnis 0,2 : 1 bis 1,2 : 1

Hydratation in der Flüssigphase bzw. gemischter Phase:
- Isopropanol:
  - 120—160° C, 30—200 bar
  - Wasser/Olefin-Verhältnis 1 : 1 bis 30 : 1
- Butanole:
  - 100—150° C, 20—100 bar
  - Wasser/Olefin-Verhältnis 1 : 1 bis 30 : 1

An folgenden Beispielen soll die Erfindung erläutert werden, wobei auf die beigefügte Zeichnung Bezug genommen wird.

## Beispiel 1

In einer Anlage zur Erzeugung von Äthanol durch Hydratation von Äthylen, die aus einem Reaktor (1) gefüllt mit 18 $m^3$ kieselsäurehaltigen Träger mit einem $H_3PO_4$-Gehalt von 38 Gew.-% besteht, zu der bei einem Synthesedruck von 70 bar und einer Synthesetemperatur von 235° C, die im Spitzenvorheizer (2) eingestellt wird, stündlich über Leitung (3) 25 470 kg Äthylen gegeben werden sowie 5420 kg Wasser, so daß ein Wasser-Olefin-Molverhältnis von 0,3 : 1 vorliegt, setzen sich stündlich 1050 kg Äthylen zu 1695 kg Äthanol um, also mit einer Ausbeute von 98%, der Umsatz des eingesetzten Äthylens beträgt 4,2%. Das umgesetzte Äthylen wird durch Frischgas aus Leitung (4) ersetzt, das Prozeßwasser wird über Leitung (5) zugegeben. Die hohe Ausbeute wird durch weitgehende Rückführung der Nebenprodukte erzielt:

Nachdem das den durch Leitung (6) den Reaktor (1) verlassende Gasgemisch im Wärmeaustauscher (7) im Gegenstrom zum kalten Umlaufgas abgekühlt und im Wascher (8) mit Waschwasser aus Leitung (9) gewaschen wurde, sind alle wasserlöslichen Reaktionsprodukte aus dem nicht umgesetzten Äthylen entfernt, und es wird über Leitung (10) mit Umlaufkompressor dem Reaktor (1) gemeinsam mit frischem Äthylen, Prozeßwasser und den zurückgeführten Nebenprodukten wieder zugeführt.

Zur Entfernung der in Wasser nicht löslichen Nebenprodukte aus dem Umlaufgas, es handelt sich dabei im wesentlichen um höher polymerisierte Kohlenwasserstoffe, wird ein Teil des Umlaufgases über Leitung (11) der Äthylen-Reinigungskolonne (12) zugeführt. Gereinigtes Äthylen geht über Kopf dieser Kolonne über Leitung (13) dem Umlaufgas wieder zu, hochsiedende Verunreinigungen werden aus dem Sumpf über Leitung (14) ausgeschleust. Bei den genannten Produktionszahlen sind diese pro Stunde jedoch nur 17 kg, was einem Ausbeuteverlust von 1,6% entspricht.

Der verdünnte Rohalkohol aus dem Wascher (8) gelangt über Leitung (15) zum Entspannungstank (16), das bei der Entspannung frei werdende gelöste Gas geht über Leitung (17) und einen nicht dargestellten Kompressor zum Umlaufgas zurück. Über Leitung (18) wird der entspannte Rohalkohol der Hydroselektionskolonne (19) zugeführt, in der die wasserdampfflüchtigen Verunreinigungen des Alkohols, also Diäthyläther, niedrigsiedende Kohlenwasserstoffe, Acetaldehyd, durch Aufgabe von Waschwasser über Kopf abgetrennt werden. Das Kopfprodukt zerfällt im Dekanteur (20) in eine wäßrige und eine organische Schicht. Die organische Schicht, etwa 46 kg mit folgender Zusammensetzung: 2,6% Kohlenwasserstoffe, 93,0% Diäthyläther, 1,2% Acetaldehyd, 0,6% Äthanol, 1,8% Butanole, 0,8% Wasser, wird über Leitung (21) dem Umlaufgas wieder zugeführt, die wäßrige Schicht enthält die Hauptmenge des Acetaldehyds, insgesamt 176 kg mit 5,3% Diäthyläther, 3,0% Acetaldehyd, 0,6% Äthanol, 0,1% Butanole, 91,0% Wasser und wird zur Ausschleusung des Acetaldehyds durch Leitung (22) abgezogen. In einer nicht dargestellten Kolonne wird Acetaldehyd als Azeotrop mit Äther über Kopf abgetrennt (7 kg mit 76,0% Acetaldehyd und 24,0% Diäthyläther), auf Alkohol bezogen ist das ein Ausbeuteverlust von 0,4%, das Sumpfprodukt wird wie die organische Schicht des Dekanteurs dem Umlaufgas zugeführt.

Der vorgereinigte verdünnte Alkohol gelangt aus der Hydroselektionskolonne (19) über Leitung (23) zur Rektifizierkolonne (24) wo über Kopf als Produkt mit Wasser azeotrop siedendes Äthanol gewonnen und über Leitung (25) abgezogen wird, während das reine Wasser als Sumpfprodukt als Waschwasser verwendet wird.

In der Mitte der Rektifizierkolonne (24) reichern sich die höher siedenden Alkohole an, die sich als Nebenprodukte gebildet haben, weil sie höher als Äthanol sieden, sich in der Wasserdampfflüchtigkeit je nach Konzentration jedoch nur gerinfügig von ihm unterscheiden. Durch Leitung (27) werden sie einer Nebenkolonne (28) zugeführt, in der sie weiter angereichert werden und über Leitung (29) bei etwa 12 kg/h mit folgender Zusammensetzung 40,2% Äthanol, 38,0% Butanole, 21,8% Wasser dem Umlaufgas wieder zugeführt werden. Das Kopfprodukt wird über Leitung (26) mit dem Produkt aus Leitung (25) vereinigt, zur vollständigen Befreiung von Wasser können beide Ströme noch absolutiert werden. Im Sumpf der Nebenkolonne wird über Leitung (30) Wasser abgezogen. Bei Betrieb der Nebenkolonne (28) in der genannten Weise werden dort keine Nebenprodukte ausbeutemindernd ausgeschleust.

## Beispiel 2

In einer Anlage zur Erzeugung von Isopropylalkohol durch Hydration von Propylen, die aus einem Reaktor (1) gefüllt mit 20,5 $m^3$ kieselsäurehaltigem Träger mit einem $H_3PO_4$-Gehalt von 25 Gew.-% besteht, zu der bei einem Synthesedruck von 40 bar und einer Synthesetemperatur von 180° C, die im Spitzenvorheizer (2) eingestellt wird, stündlich über Leitung (3) 65 000 kg Propylen gegeben werden sowie 7500 kg Wasser, so daß ein Wasser-Olefin-Molverhältnis von 0,3 : 1 vorliegt, setzen sich stündlich 2644 kg Propylen zu 3690 kg Isopropylalkohol um, also mit einer Ausbeute von 98%, der Umsatz des eingesetzten Propylens beträgt 4,1%. Das umgesetzte Propylen wird durch frisches aus Leitung (4) ersetzt, das Prozeßwasser wird über Leitung (5) zugegeben. Die hohe Ausbeute wird durch weitgehende Rückführung der Nebenprodukte erzielt:

Nachdem das den durch Leitung (6) den Reaktor (1) verlassende Gasgemisch im Wärmeaustauscher

(7) im Gegenstrom zum kalten Umlaufgas abgekühlt und im Wascher (8) mit Waschwasser aus Leitung (9) gewaschen wurde, sind alle wasserlöslichen Reaktionsprodukte aus dem nicht umgesetzten Propylen entfernt, und es wird über Leitung (10) mit Umlaufgaskompressor dem Reaktor (1) gemeinsam mit frischem Propylen, Prozeßwasser und den zurückgeführten Nebenprodukten wieder zugeführt.

Zur Entfernung der in Wasser nicht löslichen Nebenprodukte aus dem Umlaufgas, es handelt sich dabei im wesentlichen um höher polymerisierte Kohlenwasserstoffe, wird ein Teil des Umlaufgases über Leitung (11) der Propylen-Reinigungskolonne (12) zugeführt. Gereinigtes Propylen geht über Kopf dieser Kolonne über Leitung (13) dem Umlaufgas wieder zu, hochsiedende Verunreinigungen werden aus dem Sumpf über Leitung (14) ausgeschleust. Bei den genannten Produktionszahlen sind diese pro Stunde jedoch nur 47 kg, was einem Ausbeuteverlust von 1,6% entspricht.

Der verdünnte Rohalkohol aus dem Wascher (8) gelangt über Leitung (15) zum Entspannungstank (16), das bei der Entspannung frei werdende gelöste Gas geht über Leitung (17) und einen nicht dargestellten Kompressor zum Umlaufgas zurück. Über Leitung (18) wird der entspannte Rohalkohol der Hydroselektionskolonne (19) zugeführt, in der die wasserdampfflüchtigen Verunreinigungen des Alkohols, also Diisopropyläther und niedrigsiedende Kohlenwasserstoffe durch Aufgabe von Waschwasser über Kopf abgetrennt werden. Das Kopfprodukt zerfällt im Dekanteur (20) in eine wäßrige und eine organische Schicht. Die organische Schicht mit etwa folgender Zusammensetzung: 16,5% Kohlenwasserstoffe, 81,1% Diisopropyläther, 0,1% Aceton, 0,1% Isopropylalkohol, 2,2% Hexanole, wird über Leitung (21) dem Umlaufgas wieder zugeführt, die wäßrige Schicht hat etwa folgende Zusammensetzung: 2,1% Diisopropyläther, 0,1% Hexanole, 0,1% Aceton, 0,3% Isopropylalkohol, 97,4% Wasser und wird als Rückfluß auf die Hydroselektionskolonne (19) über Leitung (31) zurückgegeben. Der vorgereinigte verdünnte Alkohol gelangt aus der Hydroselektionskolonne (19) über Leitung (23) zur Rektifizierkolonne (24), wo in der Nähe des Kopfes als Produkt mit Wasser azeotrop siedender Isopropylalkohol gewonnen und über Leitung (25) abgezogen wird, während das reine Wasser als Sumpfprodukt als Waschwasser verwendet wird.

Aceton reichert sich im Kopf der Rektifizierkolonne (24) an, es wird deshalb ein Teilstrom des Rückflusses über Leitung (33) abgezogen und in einer nicht dargestellten Kolonne destilliert. Das Sumpfprodukt dieser Kolonne wird dem Rückfluß der Rektifizierkolonne wieder zugeführt, während über Kopf Aceton ausgeschleust wird. Bei den genannten Produktionszahlen fallen pro Stunde etwa 6 kg Kopfprodukt mit folgender Zusammensetzung an: 75% Aceton, 15% Isopropylalkohol, 10% Wasser, dies entspricht einem Ausbeuteverlust von 0,2%.

In der Mitte der Rektifizierkolonne (24) reichern sich die höher siedenden Alkohole (vornehmlich n-Propanol und Hexanole) an, die sich als Nebenprodukte gebildet haben, sie unterscheiden sich in der Wasserdampfflüchtigkeit ähnlich wie Aceton nur geringfügig vom Isopropylalkohol. Durch Leitung (27) werden sie einer Nebenkolonne (28) zugeführt, in der sie zusammen mit dem Wasser als Sumpfprodukt anfallen und über Leitung (32) dem Umlaufgas wieder zugeführt werden. Die Zusammensetzung ist etwa 0,9% Isopropylalkohol, 2% Hexanole, 4% n-Propanol, 93,1% Wasser. Das Kopfprodukt der Nebenkolonne (28) wird über Leitung (26) mit dem Produkt aus Leitung (25) vereinigt, zur vollständigen Befreiung von Wasser können beide Ströme noch absolutiert werden. Bei Betrieb der Nebenkolonne (28) in der genannten Weise werden dort keine Nebenprodukte ausbeutemindernd ausgeschleust.

## Beispiel 3

In einer Anlage zur Erzeugung von Äthanol durch Hydratation von Äthylen in der Art und unter den Bedingungen, wie im Beispiel 1 beschrieben, wird dem Produkt in Leitung (29) 10 kg/h eines Butanolgemisches folgender Zusammensetzung 79 Gew.-% Butanol, 6% n-Butanol, 4% Isobutanol, 1% tert. Butanol und 10% Wasser zugesetzt und wie in Beispiel 1 beschrieben, dem Umlaufgas der Synthese zugesetzt. Dabei zeigt sich, daß dann die Menge an Butanolen in dem Produkt der Leitung (18) sich nicht erhöht hat gegenüber der Butanolmenge hier vor dem Zusatz von Butanolgemisch in Strom (29).

## Beispiel 4

In einer Anlage zur Erzeugung von sek. Butanol durch Hydratation von einem Gemisch von Buten-(1) und Buten-(2) mit Wasserdampf an einem Katalysator von $H_3PO_4$, aufgetragen auf einen Träger auf Kieselsäurebasis, in einer Konzentration von 20 Gew.-% bei einer Temperatur von 195°C im Reaktoreintritt, einem Druck von 20 bar und einem Molverhältnis von Buten: Wasser von 1 : 1,05, wobei im übrigen analog Beispiel 1 und Beispiel 2 verfahren wird, wird dem Umlaufgas das in der Destillation in Leitung (21) anfallende, in Wasser nicht lösliche Kopfprodukt der Hydroselektion, im wesentlichen sek. Butyläther, und das in Leitung (29) anfallende Alkohol-Wassergemisch, das sek. Butanol, Isobutanol, n-Butanol und Oktanole enthält, zugeführt. Dadurch erhöht sich die Ausbeute an sek. Butanol von 89,5 auf 96,2%.

Beispiel 5

In einer Anlage zur Herstellung von Isopropylalkohol durch Hydratation von Propylen mit Wasser in heterogener Phase an stark saurem Kationenaustauscherharz in der $H^+$-Form ist der Reaktor (1) als Röhrenreaktor mit zwischengeschalteten Verteilerböden ausgebildet. Die einzelnen Röhrenbündel haben eine Länge von 4 m und die Rohre einen Innendurchmesser von 40 mm. Die Temperatur am Eintritt beträgt 135° C, der Druck 70 bar und das Molverhältnis Wasser : Propylen 8 : 1. Als Belastung wird eine LHSV (liquid hourly space velocity, d. h. Geschwindigkeit der flüssigen Phase im leeren Rohr) vom 1,4 m/h gewählt. Als Ionenaustauscher wird ein sulfoniertes Mischpolymerisat von Styrol und p-Divinylbenzol vom makrorektikulärem Typ (Lewatit® SP 120 der Bayer AG) eingesetzt.

Das den Reaktor (1) über Leitung (6) verlassende Reaktionsgemisch wird im Wärmetauscher (7) im Gegenstrom mit Einsatzprodukt und anschließend mit Wasser bis auf 90° C gekühlt, die organische Phase abgetrennt und im Extraktor (8) im Gegenstrom mit dem fünffachen Volumen Wasser, eingespeist via Leitung (9), von Isopropylalkohol befreit und über Leitung (10) ganz oder teilweise in den Reaktor zurückgeführt. Die wäßrigen Phasen werden über Leitung (15) der Entspannung (16) zugeführt und dann weiter, wie im Beispiel 2 beschrieben, behandelt.

Die Ausbeute an Isopropanol beträgt 99,5%, bezogen auf umgesetztes Propylen. Diese hohe Ausbeute wird erzielt, indem die Nebenprodukte im wesentlichen Diisopropyläther und Oligomere des Propylens über Leitung (13) und unerwünschte Alkohole wie 4-Methylpentanol-(2), 2-Methylpentanol-(2), n-Propanol und im geringeren Umfang Nonanole über Leitung (32) in den Reaktor (1) zurückgeführt werden.

**Patentansprüche**

1. Verfahren zur Herstellung von Alkoholen mit 2—4 C-Atomen durch katalytische Hydratation der entsprechenden Olefine an sauren Katalysatoren bei erhöhter Temperatur und erhöhtem Druck, dadurch gekennzeichnet, daß dem Einsatzprodukt vor Eintritt in den Reaktor die Nebenprodukte der Hydratation größenordnungsmäßig in der Menge zugeführt werden, in der sie beim Durchgang des Einsatzproduktes durch das Katalysatorbett entstehen und in wäßriger und organischer Phase anfallen.

2. Verfahren nach Anspruch 1, dadurch gekennzeichnet, daß bei der Hydratation von Äthylen Diäthyläther, Oligomere des Äthylens, Butanole und Hexanole rückgeführt werden.

3. Verfahren nach Anspruch 1, dadurch gekennzeichnet, daß bei der Hydratation von Propylen Diisopropyläther, Oligomere des Propylens, n-Propanol, Hexanole, Methylpentanole und Nonanole rückgeführt werden.

**Claims**

1. Process for the preparation of alcohols with 2 to 4 carbon atoms by catalytic hydration of the corresponding olefins on acid catalysts at elevated temperature and elevated pressure, characterized in that the by-products of the hydration are fed to the input product before entry into the reactor according to size in such a quantity they are formed upon the passage of the input product through the catalyst bed and are obtained in aqueous and organic phase.

2. Process according to claim 1, characterized in that the hydration of ethylene, diethyl ehters, oligomers of ethylene, butanols and hexanols are recycled.

3. Process according to claim 1, characterized in that in the hydration of propylene, diisopropyl ether, oligomers of propylene, n-propanol, hexanols, methylpentanols and nonanols are recycled.

**Revendications**

1. Procédé pour la préparation d'alcools ayant 2 à 4 atomes de carbone, par hydratation catalytique des oléfines correspondantes, sur catalyseurs acides, à température et pression élevées, procédé caractérisé en ce que les produits secondaires de l'hydratation sont recyclés dans le produit mis en oeuvre avant l'introduction de celui-ci dans le réacteur, dans une proportion qui est du même ordre de grandeur que la proportion dans laquelle ces produits secondaires se forment lors du passage du produit mis en oeuvre sur le lit de catalyseur, et sont obtenus en phase aqueuse et organique.

2. Procédé selon la revendication 1, caractérisé en ce que, dans le cas d'hydratation d'éthylène, on recycle du diéthyléther, des oligomères de l'éthylène, des butanols et des hexanols.

3. Procédé selon la revendication 1, caractérisé en ce que, dans le cas de l'hydratation de propylène, on recycle du diisopropyléther, des oligomères du propylène, du n-propanol des hexanols, des méthylpentanols et des nonanols.

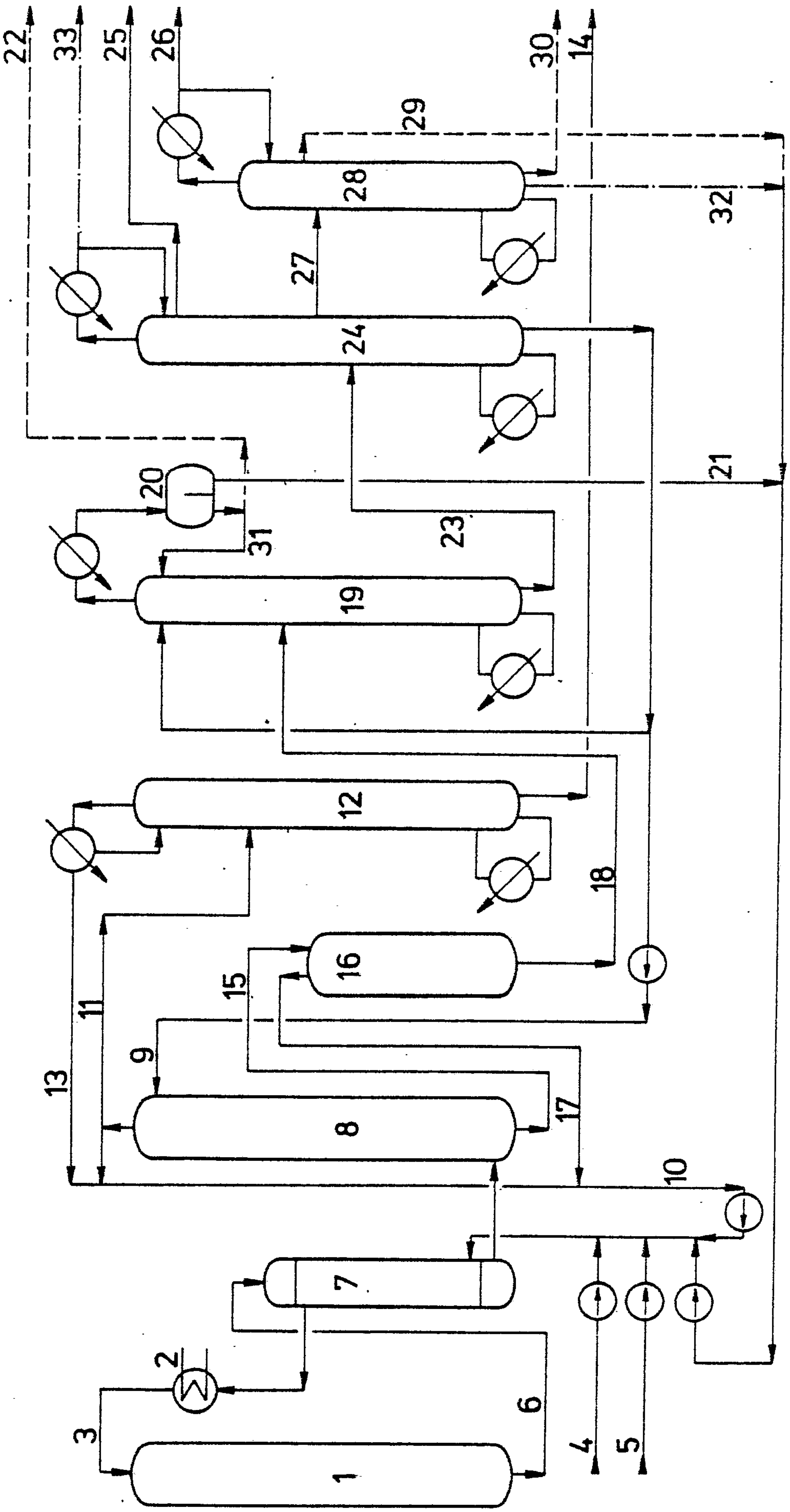
22
33
25
26
30
14
29
28
32
27
24
20
21
31
23
19
12
18
16
15
11
13
9
8
17
10
7
2
3
6
1
4
5